# EUROPÄISCHE PATENTANMELDUNG

(11) **EP 1 849 496 A2**
(43) Veröffentlichungstag der Anmeldung: **31.10.2007**
(21) Anmeldenummer: 07006350.8
(22) Anmeldetag: 28.03.2007
(51) Int. Cl.: A61N 1/37, A61N 1/372

(54) **Implantierbares batteriebetriebenes Elektrostimulationsgerät**

(30) Priorität: 25.04.2006 DE 102006019606
(71) Anmelder: BIOTRONIK CRM Patent AG, 6341 Baar (CH)
(72) Erfinder: Dörr, Thomas, 12437 Berlin (DE); Lang, Torsten, Dr., 12437 Berlin (DE)
(74) Vertreter: Lindner-Vogt, Karin L.

(57) **Zusammenfassung**

Die Erfindung betrifft ein implantierbares batteriebetriebenes Elektrostimulationsgerät (10), insbesondere zur Stimulation eines Herzens mit einer Telemetrieeinheit (11) für die drahtlose Datenübertragung zwischen Elektrostimulationsgerät (10) und einem externen (21) Gerät, einer Steuereinheit (15), die mit der Telemetrieeinheit (11) verbunden und ausgebildet ist, eine telemetrische Datenübertragung auszulösen, einer Batterie (13) zur Energieversorgung der elektrischen Komponenten des Implantates, wie der Telemetrieeinheit und der Steuereinheit, und einer Selbsttesteinheit, die ausgebildet ist, den Funktionszustand des Elektrostimulationsgerätes zu erfassen und selbsttätig akute oder drohende Fehlfunktionen zu detektieren, wobei die Selbsttesteinheit (17) mit der Steuereinheit verbunden und die Steuereinheit ausgebildet ist, im Falle einer Detektion einer akuten oder drohenden Fehlfunktion eine Datenübertragung mit Daten zum Funktionszustand des Elektrostimulationsgerätes auszulösen.

## Beschreibung

Die Erfindung betrifft ein implantierbares batteriebetriebenes Elektrostimulationsgerät, im Folgenden als Implantat bezeichnet, mit einer Telemetrieeinheit für die drahtlose Datenübertragung vom Implantat zu einem externen Gerät sowie ggf. umgekehrt.

Derartige Implantate erlauben eine telemetrische Fernüberwachung des Implantats, indem z. B. Daten aus dem Implantat über ein Service Center an einen zuständigen Arzt übermittelt werden. Die Daten können physiologische Daten sein, die das Implantat am Körper eines Patienten aufgenommen hat oder technische Daten, die den Betrieb und die Kontrolle des Implantats betreffen. Typische Implantate dieser Art sind Herzschrittmacher oder Kardioverter/Defibrillatoren.

Bei bekannten Implantaten wird eine telemetrische Datenübertragung entweder manuell (d. h. der Patient muss die Abfrage des Implantates mit anschließender Datenfernübertragung aktivieren) oder automatisch ausgelöst.

Bei batteriegespeisten Implantaten besteht grundsätzlich das Problem, dass alle Funktionen des Implantates über eine Batterie gespeist werden, die erschöpfen kann. Daher sind bereits die Therapiefunktionen des Implantats, also z. B. die Abgabe von Stimulationsimpulsen oder Defibrillationsschocks so abgestimmt, dass sie möglichst wenig Energie benötigen, ohne dass dabei eine notwendige Therapie unterbleibt. So hängt der mittlere Stromverbrauch des Implantates stark von der Therapiebedürftigkeit des Patienten ab.

Auch die Telemetriefunktionen des Implantates tragen der begrenzten Batteriekapazität Rechnung.

Bekannte manuelle Systeme lösen von sich aus keine Datenübertragung aus, also auch dann nicht, wenn ein bestimmter Betriebszustand des Implantates oder ein automatisch erkannter pathologischer Zustand des Patienten den Eingriff eines Arztes oder Technikers nahe legen. Vielmehr soll der Patient im Falle des Unwohlseins die Möglichkeit haben, durch manuelles Auslösen einer Datenübertragung von sich aus einen Arzt oder ein Service Center zu informieren, um erforderlichenfalls ärztliche Hilfe zu erfahren. Wenn der Patient die Datenübertragung ausgelöst hat, werden auch Daten betreffend den Betriebszustand des Implantats wie z.B. eine Batterieerschöpfung oder ein erkannter Gerätefehler telemetrisch dem Arzt übermittelt.

Bekannte automatische Systeme übertragen beispielsweise nach Detektion eines pathologischen Zustands automatisch die physiologischen Daten an den Arzt. Bei Erreichen der Batterieerschöpfung oder einem erkannten Gerätefehler im Rahmen eines zyklischen Selbsttests des Implantats schaltet sich dieses ab.

Der Erfindung liegt die Aufgabe zugrunde, die Betriebssicherheit der Implantate zu erhöhen.

Erfindungsgemäß wird dieses Ziel mit einem implantierbaren, batteriebetriebenen Elektrostimulationsgerät erreicht, das eine Telemetrieeinheit, eine mit der Telemetrieeinheit wenigstens mittelbar verbundene Steuereinheit, eine Batterie zur Energieversorgung sowie eine Selbsttesteinheit aufweist. Die Steuereinheit ist ausgebildet, eine telemetrische Datenübertragung über die Telemetrieeinheit auszulösen.

Die Selbsttesteinheit ist ausgebildet, den Funktionszustand des Elektrostimulationsgerätes zu erfassen und selbsttätig akute oder drohende Fehlfunktionen zu detektieren und daraufhin eine telemetrische Datenübertragung über die Telemetrieeinheit direkt oder mittels der Steuereinheit auszulösen. Selbsttesteinheit und Steuereinheit sind miteinander verbunden und entweder die Selbsttesteinheit oder die Steuereinheit löst im Falle der Detektion einer akuten oder drohenden Fehlfunktion des Implantats eine Datenübertragung mit Daten zum Funktionszustand des Elektrostimulationsgerätes aus. Die Erfahrung beruht auf der Erkenntnis, dass es insbesondere bei implantierbaren Kardiovertern/Defibrillatoren als Elektrostimulator auch zu kurzfristigen, unvorhersehbaren Batterieerschöpfungen kommen kann, wenn ein solcher Kardioverter/Defibrillator eine große Anzahl von Entladungen innerhalb eines kurzen Zeitraumes abgeben muss. Dies kann beispielsweise infolge einer häufig rezidivierenden lebensbedrohlichen Arrhythmie, die auch als electric storm bezeichnet wird, vorkommen. Da dieses Ereignis und der daraus resultierende Betriebszustand des Elektrostimulationsgerätes nicht allzu häufig vorkommt, wurde ihm möglicherweise bei herkömmlichen Elektrostimulationsgeräten keine allzu große Beachtung geschenkt. Dabei erfordert gerade das von solchen rezidivierenden lebensbedrohlichen Arrhythmien erschöpfte Elektrostimulationsgerät sowie der das Elektrostimulationsgerät tragende Patient eine baldige Aufmerksamkeit des Arztes. In diesem Zusammenhang erlaubt es das erfindungsgemäße Elektrostimulationsgerät in vorteilhafter Weise, dass der Arzt unmittelbar und automatisch von dem Elektrostimulationsgerät über dessen Betriebszustand informiert wird.

In einer bevorzugten Ausführungsvariante der Erfindung weist die Selbsttesteinheit eine Batterieüberwachungseinheit auf, die ausgebildet ist, den Erschöpfungszustand der Batterie zu erfassen und entweder direkt oder über die Steuereinheit im Falle einer kurz bevorstehenden endgültigen Erschöpfung der Batterie eine Datenübertragung mit Daten zum Erschöpfungszustand der Batterie auszulösen.

Dabei sind entweder die Batterieüberwachungseinheit selbst oder die mit ihr verbundene Steuereinheit vorzugsweise ausgebildet, einen solchen Zustand der Batterieerschöpfung zu detektieren, bei dem die verbleibende Restladung der Batterie hinreichend ist, dass die Telemetrieeinheit automatisch mindestens noch eine Nachricht mit der Information über die Batterieerschöpfung an ein externes Gerät übertragen kann. Damit ist sichergestellt, dass eine möglicherweise entscheidende, den Arzt informierende Datenübertragung mit der restlichen Batterieladung möglich ist.

Alternativ oder zusätzlich zur Batterieüberwachungseinheit weist ein bevorzugtes Elektrostimulationsgerät eine Fehlererkennungseinheit zur Erkennung von Gerätefehlern als Teil der Selbsttesteinheit auf. Diese Fehlererkennungseinheit ist entweder unmittelbar oder über die Steuereinheit mit der Telemetrieeinheit verbunden und ausgebildet, im Falle eines erkannten Gerätefehlers das Elektrostimulationsgerät in einen Sicherheitsmode umzuschalten und in diesem Sicherheitsmode automatisch mindestens eine Datenübertragung mit der Information über die Aktivierung des Sicherheitsmodes auszulösen. Ein derartiges Elektrostimulationsgerät kann nicht nur den Erschöpfungszustand seiner Batterie erfassen, sondern auch andere Gerätefehler. Darüber hinaus meldet das Elektrostimulationsgerät derartige Gerätefehler nicht nur automatisch für Datenübertragung über die Telemetrieeinheit, sondern aktiviert auch einen Sicherheitsmode, der Grundfunktionen des Elektrotherapiegerätes sicherstellt und es auf diese Weise erlaubt, dass das Elektrotherapiegerät trotz Gerätefehlers sicherheitsrelevante Grundfunktionen weiter ausführen kann.

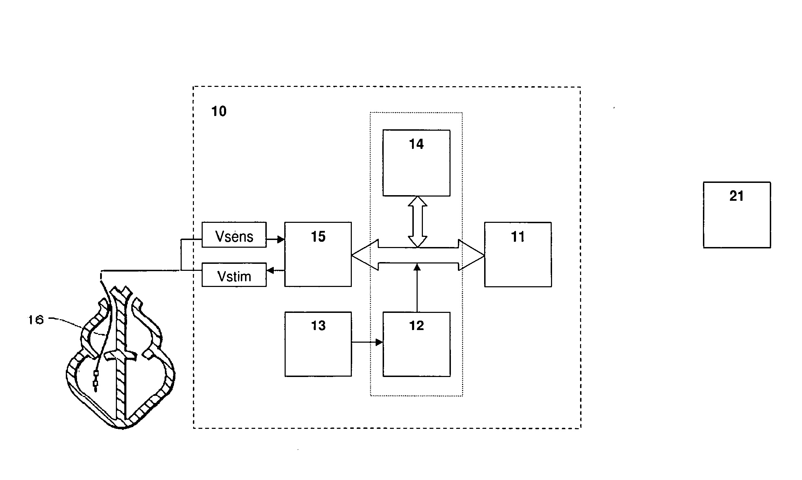

Die Erfindung soll nun anhand eines Ausführungsbeispiels mit Bezug auf die Figur näher erläutert werden:

Die Figur zeigt ein schematisches Blockschaltbild eines erfindungsgemäßen Elektrostimulationsgerätes.

In der Figur ist das Stimulationsgerät als gestrichelter Kasten 10 dargestellt. Zu erkennen ist, dass das Stimulationsgerät 10, beispielsweise ein Herzschrittmacher und Kardioverter/Defibrillator mit einer Elektrodenleitung 16 verbunden ist, die bei implantiertem Elektrotherapiergerät 10 im Beispielsfalle im rechten Ventrikel eines Herzens endet.

Diese Elektrodenleitung 16 ist im abgebildeten Beispiel über eine ventrikuläre Stimulationseinheit Vₛₜᵢₘ und eine ventrikuläre Sensingeinheit Vₛₑₙₛ mit einer Steuereinheit 15 im Inneren des Elektrostimulationsgerätes 10 verbunden.

Darüber hinaus kann das Elektrostimulationsgerät die üblichen Komponenten eines implantierbaren Herzschrittmachers oder eines implantierbaren Kardioverters/Defibrillators aufweisen, nämlich im Falle eines Zweikammerschrittmachers zusätzlich eine atriale Stimulationseinheit und eine atriale Sensingeinheit, um einerseits Stimulationsimpulse auch an das Atrium abgeben zu können und andererseits natürliche atriale Kontraktionen anhand der Auswertung elektrischer Potentialverläufe im Herzen (erfasst über ein intrakardiales Elektrokardiogramm) zu detektieren. Im Falle eines Kardioverters/Defibrillators umfasst die Steuereinheit außerdem einen Arrhythmiedetektor und einen Defibrillationsschockgenerator. In der Figur sind nur beispielhaft eine ventrikuläre Sensingeinheit Vₛₑₙₛₑ und eine ventrikuläre Stimulationseinheit Vₛₜᵢₘ abgebildet, die mit der Steuereinheit 15 verbunden sind.

Die Steuereinheit 15 ist über einen Bus mit einer Telemetrieeinheit 11 verbunden, um auf diese Weise wenigstens Daten vom Elektrostimulationsgerät 11 an ein externes Gerät 21, beispielsweise ein Patientengerät oder eine Relaisstation, zu übermitteln. Üblicherweise sind diese Daten physiologische Daten, die das Elektrostimulationsgerät 11 aufgenommen hat und zur Begutachtung durch einen Arzt via Patientengerät 21 bzw. Relaisstation 21 an ein zentrales Servicecenter übermittelt.

Eine Batterie 13 versorgt alle elektrischen Komponenten des Elektrostimulationsgerätes 11 mit elektrischer Energie. Außerdem ist die Batterie 13 mit einer Batterieüberwachungseinheit 12 verbunden, die ausgebildet ist, den Ladezustand der Batterie 13 kontinuierlich oder in regelmäßigen Abständen zu überwachen, um es auf diese Weise zu ermöglichen, den Erschöpfungszustand der Batterie 13 zu erfassen. Das Erfassen des Erschöpfungszustandes kann durch die Batterieüberwachungseinheit 12 selbst erfolgen, oder durch die Steuereinheit 15, die dazu über die Batterieüberwachungseinheit mit entsprechenden Daten versorgt wird.

Im Falle eines akuten Erschöpfungszustandes der Batterie 13 löst entweder die Batterieüberwachungseinheit 12 direkt oder die Steuereinheit 15 eine Datenübertragung über die Telemetrieeinheit 11 aus. Die übertragenden Daten enthalten ein Signal, das den Batterieerschöpfungszustand kennzeichnet und damit signalisiert, dass eine entgültige Erschöpfung der Batterie 13 kurz bevorsteht.

Die Batterieüberwachungseinheit 12 oder die Steuereinheit 15 sind zu diesem Zwecke so ausgebildet, dass sie eine Datenübertragung mit Daten zum Erschöpfungszustand der Batterie dann auslösen, wenn der Ladezustand der Batterie 13 eine solche Datenübertragung noch zulässt, also vor der endgültigen Erschöpfung. Damit ist sichergestellt, dass die verbliebene Ladung der Batterie 13 für die Telemetrieeinheit 11 ausreicht, um die Datenübertragung mit den Daten zum Erschöpfungszustand der Batterie zu ermöglichen.

Außerdem weist das Elektrostimulationsgerät 10 eine Fehlererkennungseinheit 14 zum Erkennen von Gerätefehlern auf. Die Fehlererkennungseinheit 14 ist direkt mit der Steuereinheit 15 verbunden und dazu ausgebildet, die von der Steuereinheit 15 übertragenden Daten auf Plausibilität zu überprüfen. Im Falle einer Dateninkonsistenz löst die Fehlererkennungseinheit 14 ein Fehlersignal aus, welches zum einen bewirkt, dass das Elektrostimulationsgerät 10 in einem Sicherheitsmode umgeschaltet wird, in dem ein Betrieb der wesentlichen Funktionen des Elektrostimulationsgerätes 10 von robusten Backup-Komponenten sichergestellt wird. Außerdem bewirkt das Fehlersignal eine Datenübertragung über die Telemetrieeinheit 11. Bei dieser Datenübertragung überträgt die Telemetrieeinheit 11 ein Signal, das das Umschalten des Elektrostimulationsgerätes 10 in den Sicherheitsmode kennzeichnet. Auf diese Weise ist das Elektrostimulationsgerät 10 in der Lage, automatisch und selbsttätig beispielsweise einen behandelnden Arzt darüber zu informieren, dass das Elektrostimulationsgerät 10 im Rahmen eines Selbsttests durch die Fehlererkennungseinheit 14 einen Gerätefehler erkannt hat und sich in den Sicherheitsmode umgeschaltet hat.

Die Batterieüberwachungseinheit 12 und die Fehlererkennungseinheit 14 bilden auf diese Weise eine Selbsttesteinheit 17, die dazu ausgebildet ist, regelmäßig den Betriebszustand des Elektrostimulationsgerätes 10 zu erfassen, und zwar insbesondere hinsichtlich möglicher Gerätefehler und hinsichtlich des Ladungszustandes bzw. Erschöpfungszustandes der Batterie 13. Die Selbsttesteinheit löst im Falle eines erkannten Gerätefehlers oder im Falle eines erkannten Erschöpfungszustandes der Batterie direkt oder mittels der Steuereinheit 15 eine entsprechende Datenübertragung über die Telemetrieeinheit 11 aus. Bei dieser Datenübertragung werden Informationen über den Gerätezustand des Elektrostimulationsgerätes 10 automatisch übermittelt.

## Patentansprüche

1. Implantierbares batteriebetriebenes Elektrostimulationsgerät (10), insbesondere zur Stimulation eines Herzens mit
- einer Telemetrieeinheit (11) für die drahtlose Datenübertragung zwischen Elektrostimulationsgerät (10) und einem externen (21) Gerät,
- einer Steuereinheit (15), die mit der Telemetrieeinheit (11) verbunden und ausgebildet ist, eine telemetrische Datenübertragung auszulösen,
- einer Batterie (13) zur Energieversorgung der elektrischen Komponenten des Implantates, wie der Telemetrieeinheit und der Steuereinheit, und
- einer Selbsttesteinheit, die ausgebildet ist, den Funktionszustand des Elektrostimulationsgerätes zu erfassen und selbsttätig akute oder drohende Fehlfunktionen zu detektieren,
**dadurch gekennzeichnet, dass** die Selbsttesteinheit (17) mit der Steuereinheit verbunden und ausgebildet ist, im Falle einer Detektion einer akuten oder drohenden Fehlfunktion eine Datenübertragung mit Daten zum Funktionszustand des Elektrostimulationsgerätes auszulösen.

2. Implantierbares Elektrostimulationsgerät (10) nach Anspruch 1,
**dadurch gekennzeichnet, dass** die Selbsttesteinheit eine Batterieüberwachungseinheit (12) aufweist, die ausgebildet ist, den Erschöpfungszustand der Batterie zu erfassen und im Falle einer kurz bevorstehenden endgültigen Erschöpfung der Batterie direkt oder über die Steuereinheit eine Datenübertragung mit Daten zum Erschöpfungszustand der Batterie auszulösen.

3. lmplantierbares Elektrostimulationsgerät (10) nach Anspruch 2,
**dadurch gekennzeichnet, dass** die Batterieüberwachungseinheit oder die Steuereinheit ausgebildet ist, einen Zustand der Batterieerschöpfung zu detektieren, bei dem die verbleibende Restladung der Batterie (13) hinreichend ist, dass die Telemetrieeinheit automatisch mindestens noch eine Nachricht mit der Information über die Batterieerschöpfung an ein externes Gerät (21) übertragen kann, um die Datenübertragung bei Erreichen dieses Erschöpfungszustandes der Batterie (13) auszulösen.

4. Implantierbares Elektrostimulationsgerät (10) nach Anspruch 1, **dadurch gekennzeichnet, dass** die Selbsttesteinheit (17) eine Fehlererkennungseinheit (14) zur Erkennung von Gerätefehlern aufweist, die mit der Steuereinheit (15) verbunden ist, und dass entweder die Fehlererkennungseinheit (14) oder die Steuereinheit (15) ausgebildet ist,
- im Falle eines erkannten Gerätefehlers das Elektrostimulationsgerät in einen Sicherheitsmode umzuschalten und
- im Sicherheitsmode automatisch mindestens eine Datenübertragung mit der Information über die Aktivierung des Sicherheitsmodes auszulösen.
